# EUROPÄISCHE PATENTSCHRIFT

(11) **EP 0 903 410 B1**
(45) Veröffentlichungstag und Bekanntmachung des Hinweises auf die Patenterteilung: **12.05.2004**
(21) Anmeldenummer: 98115696.1
(22) Anmeldetag: 20.08.1998
(51) Int. Cl.: C12Q 1/28, C12Q 1/44

(54) **Verfahren zum Nachweis und zur Bestimmung enzymatischer Reaktionen in Urin**
Method for detection and determination of enzymatic reactions in urine
Procédé pour la détection et la détermination des réactions enzymatiques dans l'urine

(30) Priorität: 19.09.1997 DE 19741447
(43) Veröffentlichungstag der Anmeldung: 24.03.1999
(73) Patentinhaber: Dade Behring Marburg GmbH, 35001 Marburg (DE)
(72) Erfinder: Schmitz-Treyer, Ursula, 80336 München (DE); Hofmann, Walter, Dr., 82008 Unterhaching (DE); Guder, Walter, Prof. Dr., 81673 München (DE)

(56) Entgegenhaltungen:
- EP-A1- 0 094 554
- EP-A1- 0 597 410
- EP-A2- 0 230 229
- EP-A2- 0 279 614
- US-A- 5 135 875
- US-A- 5 516 700

## Beschreibung

Die vorliegende Erfindung betrifft ein Verfahren zum Nachweis und zur Bestimmung enzymatischer Reaktionen in Urin, wobei der Reaktion bestimmte Substanzen zugesetzt werden, die die optische Meßbarkeit der enzymatischen Reaktion verbessern.

In der Diagnostik der Erkrankung der Nieren und des Urogenitaltraktes hat der Nachweis bestimmter Enzyme, insbesondere von Pseudoperoxidasen und Esterasen in Körperflüssigkeiten, insbesondere im Urin, große Bedeutung. Ursprünglich spielten in der Urindiagnostik physikalische Methoden, wie z. B. Abzentrifugieren und Auszählen von Leukozyten eine besondere Rolle. Diese Verfahren - obwohl vom Ergebnis her befriedigend - sind heutzutage nicht mehr praktikabel, da sie ein hohes Maß an manueller Tätigkeit verlangen. Auch die im Bereich der Urindiagnostik sehr weit verbreiteten sogenannten 'Teststreifen" erfordern ein gewisses Maß an Manipulation, so daß eine Automatisierung der Messung von der Probenahme bis zur Auswertung kaum möglich ist. Besonderer Bedeutung im Rahmen der Urindiagnostik kommt zum einen der Erfassung der Granulozytenesterasen als eine Basisuntersuchung im Rahmen des Harnstatus sowie der Erfassung von Blut im Urin mittels der Bestimmung von Pseudoperoxidasen zu.

Die Messung dieser Parameter mittels Teststreifen hat sich in der Praxis unter anderem deswegen lange halten können, weil Teststreifen aufgrund der chromatographischen Eigenschaften des Trägermaterials die Messung wesentlich vereinfachen. Entsprechende Verfahren und Mittel dazu sind bereits beschrieben.

US5516700 offenbart ein naßchemisches Verfahren zum Nachweis und zur Bestimmung von Analyten im Urin.

Aufgabe der Erfindung war s daher, ein naßchemisches Verfahren zur Bestimmung bestimmter Enzyme in Urin zu entwickeln. Überraschenderweise hat sich gezeigt, daß durch die Verwendung bestimmter Reagenzienzusätze die optische Bestimmung von Enzymen auch in gefärbten Urinen sich leicht an automatisierten Analysegeräten durchführen läßt.

Beschrieben wird ein Verfahren zum Nachweis und zur Bestimmung enzymatischer Reaktionen in Urin, in dem eine die nachzuweisenden Enzyme enthaltende Urinprobe in einem automatisierten Analysengerät mit einem spezifischen Substrat versetzt wird, die enzymatische Reaktion optisch verfolgt und gemessen wird, und der Reaktion eine die Meßbarkeit verbessernde Substanz zugesetzt wird, wobei diese Substanz ausgewählt wird aus der Gruppe der niederen Alkohole: Methanol, Ethanol und Propanol.

Vorteilhafterweise wird dabei der Alkohol in einer Konzentration von etwa 1 bis 70 Vol.% - im Reaktionsansatz - eingesetzt, bevorzugterweise etwa 10 bis 60 Vol %, besonders bevorzugterweise 40 bis 60 Vol %, ganz besonders bevorzugterweise 45 bis 55 Vol. %.

Vorteilhafterweise ist das nachzuweisende Enzym die Hämoglobinpseudoperoxidase oder Leukozytenesterase.

Zum Nachweis der Reaktion der Hämoglobinpseudoperoxidase wird vorteilhafterweise TMB eingesetzt und mindestens eine Substanz aus der folgenden Gruppe dem Reaktionsansatz zugesetzt :
6-Methoxyquinolin und Phenanthridin, wobei 6-Methoxyquinolin in einer Konzentration von 0,1 bis 0,5 Vol.% und Phenanthridin in einer Konzentration von 1 bis 3 mg/ml zugesetzt wird.

Um die Leukozytenesterase nachzuweisen, wird als Substrat der an sich bekannte 3-Hydroxy-5-Phenyl-pyrrol-N-tosyl-L-alaninester eingesetzt, ein Diazoniumsalz und mindestens eine Substanz aus der folgenden Gruppe dem Reaktionsansatz zugesetzt: Brij®, Cholsäure, Ethylenglykol und EDTA.

Das Diazoniumsalz ist vorteilhafterweise Diazoniumchloridzinkat und wird in einer Konzentration von 0,02 bis 0,1 mg/ml zugesetzt, Brij® in einer Konzentration von 0,1 bis 0,5 Vol.%, Cholsäure in einer Konzentration von 0,02 bis 0,3 Vol.%, Ethylenglykol in einer Konzentration von 0,1 bis 0,5 Vol.% und EDTA in einer Konzentration von 0,1 bis 0,5 mmol/l.

Zur Vermeidung von Ascorbinsäurestörungen kann Ascorbatoxidase zugesetzt werden.

Vorteilhafterweise wird eine Doppelbestimmung gemacht, wobei nur bei einer der Bestimmungen das Substrat zugegeben wird und als Meßwert die Differenz aus der Bestimmung ohne und der Bestimmung mit Substrat gewertet wird.

Verwendete Abkürzungen:
- EDTA: Äthylendiamintetraessigsäure
- PBS: Phosphatgepufferte Kochsalzlösung
- TMB: Tetramethylbenzidin

Die folgenden Beispiele sollen die Erfindung erläutern.

### Beispiel 1

### Naßchemische Messung von Leukozytenesterase im Urin

Die Bestimmung erfolgte an einem Hitachi 911 (Boehringer Mannheim, Deutschland). Es werden die folgenden Reagenzien eingesetzt:
- R1:: 0.25 mol/l PBS, pH 7.5
0.5 % Brij®
1.0 % Ethylenglykol
0.25 % Cholsäure
1 mmol/l EDTA
- R2:: 2.5 mg 3-Hydroxy-5-phenyl-pyrrol-N-tosyl-L-alanineester in
9.8 ml Ethanol (96 %) + 0.2 ml Decanol
- R3:: 0.625 mg/ml Diazoniumchloridzinkat in Aqua dest.
- R4:: 70 ml Ethanol (96 %) + 30 ml Aqua dest.

Die Messung erfolgt als Endpunktbestimmung bei 546 nm. Die Reagenzvolumen betragen:
- R1:: 40 µl
- R2:: 20 µl
- R3:: 20 µl
- R4:: 160 µl
Probe: 40 µl unverdünnter Urin

### Beispiel 2

### Naßchemische Messung von Hämoglobinpseudoperoxidase im Urin

Die Bestimmung erfolgte an einem Hitachi 911 (Boehringer Mannheim, Deutschland). Die Messung erfolgte als Endpunktbestimmung bei 660 nm. Es wurden die folgenden Reagenzien eingesetzt:
- R1:: 0.1 mg/ml Ascorbatoxidase in 0.1 mol/l phosphatgepufferter NaCl-Lösung (PBS)
- R2:: 1.0 mol/l Citratpuffer pH 4.8
- R3:: 45 mg TMB
+ 4 ml R2
+ 6 ml Ethanol (96 %)
+ 0.4 ml 6-Methoxyquinoline
+ 280 mg Phenanthridine
+ 200 mg Dihydroperoxid (Luperox®)
- R4:: 70 ml Ethanol (96 %) + 30 ml Aqua dest.

Die Reagenzienvolumina betrugen:
- R1: 20 µl
- R2: 1µl
- R3: 20 µl
- R4: 190 µl
Probe: 50 µl unverdünnter Urin

## Patentansprüche

1. Naßchemisches Verfahren zum Nachweis und zur Bestimmung enzymatischer Reaktionen in Urin, wobei
a) eine das nachzuweisende Enzym enthaltende Urinprobe in einem automatisierten Analysengerät mit einem spezifischen Substrat versetzt wird,
b) die enzymatische Reaktion optisch verfolgt und gemessen wird, und
c) der Reaktion eine die Meßbarkeit verbessernde Substanz zugesetzt wird,
**dadurch gekennzeichnet, daß** besagtes Substrat in flüssiger Form zugesetzt wird daß besagte Substanz ausgewählt wird aus der Gruppe der niederen Alkohole: Methanol, Ethanol und Propanol.

2. Verfahren gemäß Anspruch 1), **dadurch gekennzeichnet, daß** der Alkohol in einer Konzentration von etwa 1 bis 70 Vol.% - im Reaktionsansatz - eingesetzt wird.

3. Verfahren gemäß einem der Ansprüche 1 bis 2), **dadurch gekennzeichnet, daß** das nachzuweisende Enzym die Hämoglobinpseudoperoxidase ist.

4. Verfahren gemäß Anspruch 3) zur Bestimmung von Blut in Urin

5. Verfahren gemäß einem der Ansprüche 1 bis 2), **dadurch gekennzeichnet, daß** das nachzuweisende Enzym die Leukozytenesterase ist.

6. Verfahren gemäß Anspruch 3), **dadurch gekennzeichnet, daß** zum Nachweis der Reaktion Tetramethylbenzidin (TMB) eingesetzt wird und mindestens eine Substanz aus der folgenden Gruppe dem Reaktionsansatz zugesetzt wird: 6-Methoxyquinolin und Phenanthridin.

7. Verfahren gemäß Anspruch 6), **dadurch gekennzeichnet, daß** 6-Methoxyquinolin in einer Konzentration von 0,1 bis 0,5 Vol.% und Phenanthridin in einer Konzentration von 1 bis 3 mg/ml zugesetzt wird.

8. Verfahren gemäß Anspruch 5), **dadurch gekennzeichnet, daß** als Substrat 3-Hydroxi-5-Phenyl-pyrrol-N-tosyl-L-alaninester eingesetzt wird, ein Diazoniumsalz und mindestens eine Substanz aus der folgenden Gruppe dem Reaktionsansatz zugesetzt wird:
Cholsäure, Ethylenglykol und EDTA.

9. Verfahren gemäß Anspruch 8), **dadurch gekennzeichnet, daß** das Diazoniumsalz Diazoniumchloridzinkat ist und in einer Konzentration von 0,02 bis 0,1 mg/ml zugesetzt wird. Cholsäure in einer Konzentration von 0,02 bis 0,3 Vol.%, Ethylenglykol in einer Konzentration von 0,1 bis 0,5 Vol.% und EDTA in einer Konzentration von 0,1 bis 0,5 mmol/l zugesetzt werden.

10. Verfahren gemäß einem der Ansprüche 1 bis 9), **dadurch gekennzeichnet, daß** zur Vermeidung von Ascorbinsäurestörungen Ascorbatoxidase zugesetzt wird.

11. Verfahren gemäß einem der Ansprüche 1 bis 10), **dadurch gekennzeichnet, daß** eine Doppelbestimmung gemacht wird, wobei nur bei einer der Bestimmungen das Substrat zugegeben wird und als Meßwert die Differenz aus der Bestimmung ohne und der Bestimmung mit Substrat gewertet wird.

## Claims

1. A wet-chemical method for the detection and for the determination of enzymatic reactions in urine, in which
a) a urine sample containing the enzyme to be detected is mixed with a specific substrate in an automatic analyzer,
b) the enzymatic reaction is followed and measured optically, and
c) a substance improving the measurability is added to the reaction,
wherein said substrate is added in liquid form and said substance is selected from the group of lower alcohols: methanol, ethanol and propanol.

2. The method as claimed in claim 1, wherein the alcohol is employed in a concentration of about 1 to 70% by volume - in the reaction mixture.

3. The method as claimed in either of claims 1 and 2, wherein the enzyme to be detected is hemoglobin pseudoperoxidase.

4. The method as claimed in claim 3 for the determination of blood in urine.

5. The method as claimed in either of claims 1 and 2, wherein the enzyme to be detected is leukocyte esterase.

6. The method as claimed in claim 3, wherein tetramethylbenzidine (TMB) is employed to detect the reaction, and at least one substance from the following group is added to the reaction mixture: 6-methoxyquinoline and phenanthridine.

7. The method as claimed in claim 6, wherein there is addition of 6-methoxyquinoline in a concentration of 0.1 to 0.5% by volume and phenanthridine in a concentration of 1 to 3 mg/ml.

8. The method as claimed in claim 5, wherein 3-hydroxy-5-phenylpyrrole N--tosyl-L-alanine ester is employed as substrate, and a diazonium salt and at least one substance from the following group are added to the reaction mixture: cholic acid, ethylene glycol and EDTA.

9. The method as claimed in claim 8, wherein the diazonium salt is diazonium chloride zincate and is added in a concentration of 0.02 to 0.1 mg/ml, and there is addition of cholic acid in a concentration of 0.02 to 0.3% by volume, ethylene glycol in a concentration of 0.1 to 0.5% by volume and EDTA in a concentration of 0.1 to 0.5 mmol/l.

10. The method as claimed in any of claims 1 to 9, wherein ascorbate oxidase is added to avoid interference by ascorbic acid.

11. The method as claimed in any of claims 1 to 10, wherein a double determination is carried out, with the substrate being added in only one of the determinations, and the difference between the determination without and the determination with substrate being regarded as the measurement.

## Revendications

1. Procédé chimique en milieu humide pour la détection et la détermination des réactions enzymatiques dans l'urine, dans lequel
a) un échantillon d'urine contenant l'enzyme à détecter est transféré dans un analyseur automatisé avec un substrat spécifique,
b) la réaction enzymatique est observée et mesurée optiquement, et
c) une substance améliorant la mesurabilité est ajoutée à la réaction,
**caractérisé en ce que** ledit substrat est ajouté sous forme liquide et **en ce que** ladite substance est choisie dans le groupe des alcools faibles : méthanol, éthanol et propanol.

2. Procédé selon la revendication 1), **caractérisé en ce que** l'alcool est utilisé - dans le mélange réactionnel- en une concentration d'environ 1 à 70 % en volume.

3. Procédé selon une des revendications 1 à 2), **caractérisé en ce que** l'enzyme à détecter est la pseudo-peroxydase de l'hémoglobine.

4. Procédé selon la revendication 3) pour la détermination de sang dans l'urine.

5. Procédé selon une des revendications 1 à 2), **caractérisé en ce que** l'enzyme à détecter est la leucocyte estérase.

6. Procédé selon la revendication 3), **caractérisé en ce que** de la tétra-méthyl-benzidine (TMB) est utilisée pour la détection de la réaction, et **en ce qu'**au moins une substance du groupe suivant est ajoutée au mélange réactionnel : 6-méthoxyquinoline et phénanthridine.

7. Procédé selon la revendication 6), **caractérisé en ce que** la 6-méthoxyquinoline est ajoutée en une concentration de 0,1 à 0,5 % en volume, et la phénanthridine en une concentration de 1 à 3 mg/ml.

8. Procédé selon la revendication 5), **caractérisé en ce que** du 3-hydroxy-5-phényl-pyrrol-N-tosyl-L-alanine ester est utilisé comme substrat, et qu'un sel de diazonium et au moins une substance du groupe suivant sont ajoutés au mélange réactionnel : acide cholique, éthylène glycol et EDTA

9. Procédé selon la revendication 8, **caractérisé en ce que** le sel de diazonium est du chlorure de diazonium zincate et est ajouté en une concentration de 0,02 à 0,1 mg/ml, l'acide cholique est ajouté en une concentration de 0,02 à 0,3 % en volume, l'éthylène glycol en une concentration de 0,1 à 0,5 % en volume, et l'EDTA en une concentration de 0,1 à 0,5 mmol/l.

10. Procédé selon une des revendications 1 à 9), **caractérisé en ce que** de l'ascorbate oxydase est ajouté pour éviter les interférences de l'acide ascorbique.

11. Procédé selon une des revendications 1 à 10), **caractérisé en ce que** l'on effectue une double détermination, le substrat n'étant ajouté que pour l'une des déterminations, et la valeur mesurée évaluée étant la différence entre la détermination avec et la détermination sans le substrat.
